# EUROPÄISCHE PATENTANMELDUNG

(11) **EP 1 889 567 A2**
(43) Veröffentlichungstag der Anmeldung: **20.02.2008**
(21) Anmeldenummer: 07013850.8
(22) Anmeldetag: 14.07.2007
(51) Int. Cl.: A61B 3/12, A61B 3/13

(54) **Ophthalmoskopie-Vorsatzmodul für ein Operationsmikroskop**

(30) Priorität: 18.08.2006 DE 102006038911
(71) Anmelder: Carl Zeiss Surgical GmbH, 73447 Oberkochen (DE)
(72) Erfinder: Obrebski, Andreas, 73447 Oberkochen (DE)
(74) Vertreter: Gauss, Nikolai

(57) **Zusammenfassung**

Die Erfindung betrifft ein Ophthalmoskopie-Vorsatzmodul 150 zur Befestigung an einem Operationsmikroskop 100. Das Ophthalmoskopie-Vorsatzmodul 150 hat eine Halteeinrichtung 153 für eine Ophthalmoskopierlupe 154. Diese Halteeinrichtung 153 trägt eine Einrichtung 180, 181 zum Führen von Beleuchtungslicht 125. Erfindungsgemäß führt die Einrichtung 180, 181 zum Führen von Beleuchtungslicht an der Ophthalmoskopierlupe 154 vorbei Beleuchtungslicht 125 zu einem Objektbereich 190.

## Beschreibung

Die Erfindung betrifft ein Ophthalmoskopie-Vorsatzmodul zur Befestigung an einem Operationsmikroskop mit einer Halteeinrichtung für ein Ophthalmoskopierlupe, wobei die Halteeinrichtung eine Einrichtung zum Führen von Beleuchtungslicht trägt.

Ein Operationsmikroskop mit einem Ophthalmoskopie-Vorsatzmodul der eingangs genannten Art ist aus der DE 94 15 219 U1 bekannt. Dort ist als Ophthalmoskopie-Vorsatzmodul eine Vorsatzeinrichtung für ein stereoskopisches Operationsmikroskop beschrieben, das dazu dient, den Augenhintergrund eines Patientenauges zu beobachten. Die Vorsatzeinrichtung umfasst eine Ophthalmoskopierlupe in Form einer Vorsatzlinse. Sie ist in einer Halteeinrichtung mit dem Ophthalmoskopie-Vorsatzmodul unmittelbar über einem zu untersuchenden Patientenauge aufgenommen. Die Ophthalmoskopierlupe erzeugt auf ihrer dem zu untersuchenden Objekt abgewandten Seite ein Zwischenbild des Augenhintergrunds. Dieses Zwischenbild kann mit dem Operationsmikroskop, an welches das Ophthalmoskopie-Vorsatzmodul angeschlossen ist, betrachtet werden. Das Ophthalmoskopie-Vorsatzmodul weist ein Gelenk für die Halteeinrichtung der Ophthalmoskopierlupe auf. Die Ophthalmoskopierlupe kann an diesem Gelenk in und aus dem Beobachtungsstrahlengang des entsprechenden Operationsmikroskops geschwenkt werden.

Es ist möglich, das in der DE 94 15 219 U1 beschriebene Ophthalmoskopie-Vorsatzmodul an Operationsmikroskopen einzusetzen, bei denen durch das Mikroskop-Hauptobjektiv hindurch Beleuchtungslicht für den Objektbereich bereitgestellt wird. Ein solches Operationsmikroskop ist beispielsweise das OPMI^{®} Visu 200 der Firma Carl Zeiss. Ist bei diesem Operationsmikroskop die Ophthalmoskopierlupe vor dem Mikroskop-Hauptobjektiv in den stereoskopischen Beobachtungsstrahlengang geschwenkt, so gelangt das Beleuchtungslicht durch die Ophthalmoskopierlupe hindurch in das Patientenauge und beleuchtet den Augenhintergrund.

Wird der Beleuchtungsstrahlengang durch die Ophthalmoskopierlupe geführt, so können sich im Beobachtungsbild des Operationsmikroskops störende Lichtreflexe ergeben, die von Beleuchtungslicht herrühren, das an der Ophthalmoskopierlupe reflektiert wird. Solche Lichtreflexe sind umso ausgeprägter, wenn die Ophthalmoskopierlupe verschmutzt ist, etwa durch Bluttröpfchen. Eine Verschmutzung der Ophthalmoskopierlupe im laufenden Operationsbetrieb lässt sich jedoch nicht immer ausschließen.

Aufgabe der Erfindung ist es daher, ein Ophthalmoskopie-Vorsatzmodul zu schaffen, das sich zum Arbeiten mit einem Operationsmikroskop eignet, bei dem Beleuchtungslicht durch das Mikroskop-Hauptobjektiv hindurch bereitgestellt wird, und bei dem dennoch keine störenden Reflexe auftreten, die von Streuungen von Beleuchtungslicht an einer Ophthalmoskopierlupe herrühren.

Diese Aufgabe wird durch ein Ophthalmoskopie-Vorsatzmodul der eingangs genannten Art gelöst, bei dem die Einrichtung zum Führen von Beleuchtungslicht an der Ophthalmoskopierlupe vorbei Beleuchtungslicht zum Objektbereich führt.

Auf diese Weise ist es möglich, den Beleuchtungsstrahlengang gut auf ein zu untersuchendes Objekt wie ein Patientenauge abzustimmen. Insbesondere wird so gewährleistet, dass sich die Stellung der Ophthalmoskopierlupe nicht auf den Einfallswinkel von Beleuchtungslicht auswirkt, das in den zu untersuchenden Objektbereich geführt wird.

In Weiterbildung der Erfindung umfasst die Einrichtung zum Führen von Beleuchtungslicht wenigstens teilweise verstellbare optische Elemente. Auf diese Weise kann der Einfallswinkel für Beleuchtungslicht auf ein mittels des Ophthalmoskopie-Vorsatzmodul untersuchtes Patientenauge eingestellt werden. So ist es möglich, für den Beleuchtungsstrahlengang, der in den Objektbereich gelangt, einen Verlauf einzustellen, der für die Untersuchung eines Objektbereichs günstig ist.

In Weiterbildung der Erfindung sind den wenigstens teilweise verstellbaren optischen Elementen Antriebe zugeordnet. Auf diese Weise ist es möglich, eine automatisierte Einstellung günstiger Beleuchtungskonfigurationen bei dem Ophthalmoskopie-Vorsatzmodul vorzusehen, so dass dieses leicht und ergonomisch günstig bedient werden kann. Darüber hinaus ermöglichen es Antriebe für die wenigstens teilweise verstellbaren optischen Elemente einen Regelkreis vorzusehen, der das Bild eines Beobachtungsbereiches auswertet und bei dem Ophthalmoskopie-Vorsatzmodul den Verlauf eines Beleuchtungsstrahlenganges gegebenenfalls bei laufendem Operationsbetrieb für ein besonders kontrastreiches oder lichtstarkes Beobachtungsbild optimiert.

In Weiterbildung der Erfindung umfasst die Einrichtung zum Führen von Beleuchtungslicht ein oder mehrere Spiegel, ein oder mehrere Lichtleiter, ein oder mehrere Linsenelemente oder ein Ablenkprisma. Auf diese Weise ist es möglich, Beleuchtungslicht ohne Streuverluste zu einem Objektbereich zu führen, der untersucht werden soll.

In Weiterbildung der Erfindung ist in das Ophthalmoskopie-Vorsatzmodul eine Lichtquelle integriert. Auf diese Weise kann das Ophthalmoskopie-Vorsatzmodul bei Operationsmikroskopen eingesetzt werden, in denen keine Beleuchtungseinheit vorgesehen ist, die Beleuchtungslicht abgibt.

In Weiterbildung der Erfindung ist bei dem Ophthalmoskopie-Vorsatzmodul eine Beleuchtungslicht-Einkoppeleinheit vorgesehen, um von dem Operationsmikroskop bereitgestelltes Beleuchtungslicht aufzunehmen. Vorzugsweise ist die Beleuchtungslicht-Einkoppeleinheit zur Aufnahme von Beleuchtungslicht ausgelegt, welches das Mikroskop-Hauptobjektiv des Operationsmikroskops durchsetzt.

Ein Operationsmikroskop mit einem entsprechenden Ophthalmoskopie-Vorsatzmodul ermöglicht eine von störenden Reflexen befreite Beobachtung des Augenhintergrunds eines Patientenauges.

Vorteilhafte Ausführungsformen der Erfindung sind in den Zeichnungen dargestellt und werden nachfolgend beschrieben.

Es zeigen:
- Fig. 1: ein Operationsmikroskop mit einem Ophthalmoskopie-Vorsatzmodul, in das zum Führen des Beleuchtungsstrahlenganges ein Ablenkprisma integriert ist;
- Fig. 2: einen Schnitt des Ophthalmoskopie-Vorsatzmoduls aus Fig. 1 entlang der Linie II - II;
- Fig. 3: ein Operationsmikroskop mit Ophthalmoskopie-Vorsatzmodul, in das zum Führen des Beleuchtungsstrahlenganges ein Lichtleiter vorgesehen ist;
- Fig. 4: ein Operationsmikroskop mit einer alternativen Ausführungsform für ein Ophthalmoskopie-Vorsatzmodul mit einem Lichtleiter im Beleuchtungsstrahlengang, um Beleuchtungslicht zum Objektbereich zu führen;
- Fig. 5: ein Operationsmikroskop mit Ophthalmoskopie-Vorsatzmodül, welches zum Führen von Beleuchtungslicht ein Spiegelsystem hat;
- Fig. 6: ein Operationsmikroskop mit einer alternativen Ausführungsform für ein Ophthalmoskopie-Vorsatzmodul, bei dem zum Führen von Beleuchtungslicht ein Spiegelsystem vorgesehen ist; und
- Fig. 7: ein Operationsmikroskop mit Ophthalmoskopie-Vorsatzmodul, das eine integrierte Lichtquelle aufweist.

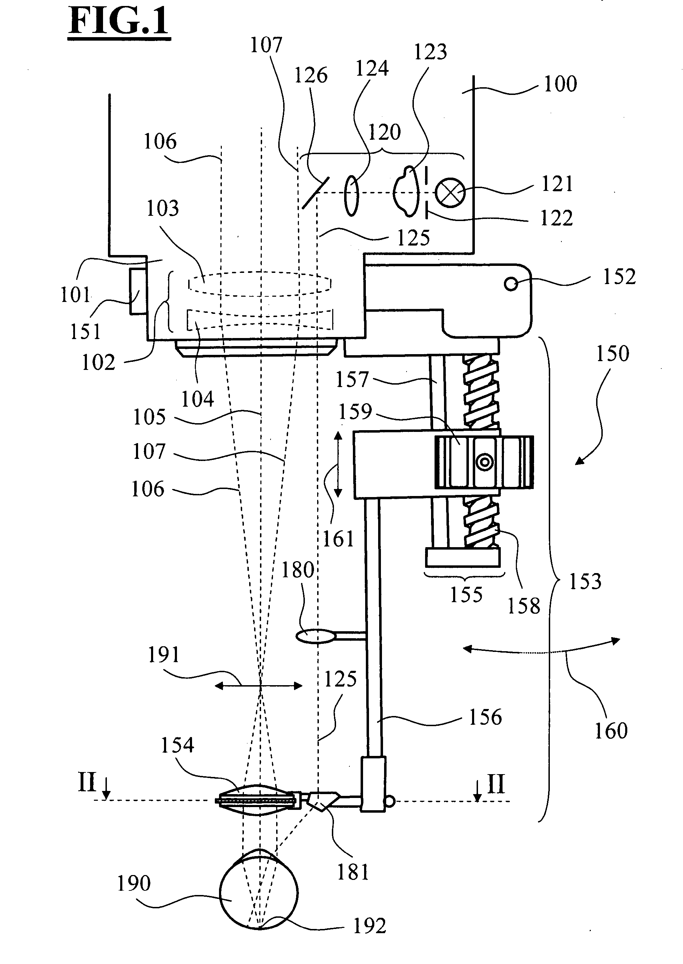

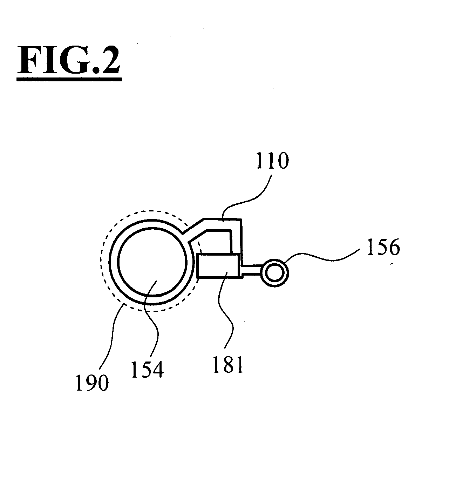

In Fig. 1 ist ein Operationsmikroskop 100 gezeigt, an welches ein Ophthalmoskopie-Vorsatzmodul 150 angeschlossen ist. Das Ophthalmoskopie-Vorsatzmodul 150 ist mit einer Halteeinrichtung 151 an einer Aufnahme 101 für ein fokussierbares Mikroskop-Hauptobjektivsystem 102 angeordnet. Das Mikroskop-Hauptobjektivsystem 102 umfasst eine Linse 103 mit positiver Brechkraft und eine Linse 104, deren Brechkraft negativ ist. Die Linse 104 kann relativ zu der Linse 103 bewegt werden, um die Fokusebene des Operationsmikroskops 100 einzustellen. Das Mikroskop-Hauptobjektivsystem 102 hat eine optische Achse 105 und wird von einem stereoskopischen Beobachtungsstrahlengang 106, 107 durchsetzt, der in dem Operationsmikroskop 100 durch ein nicht weiter dargestelltes Vergrößerungssystem geführt ist, um in einem in Fig. 2 ebenfalls nicht gezeigten Okulareinblick das Betrachten eines Objektbereichs zu ermöglichen.

Das Ophthalmoskopie-Vorsatzmodul 150 hat ein Gelenk 152. In dem Gelenk 152 ist eine Halteeinrichtung 153 mit Ophthalmoskopierlupe 154 aufgenommen. Die Halteeinrichtung 153 mit der Ophthalmoskopierlupe 154 kann in dem Gelenk 152 entsprechend dem Doppelpfeil 160 in und aus dem Beobachtungsstrahlengang 106, 107 des Operationsmikroskops 100 geschwenkt werden.

Die Halteeinrichtung 153 hat einen ersten Abschnitt 155 und einen zweiten Abschnitt 156. Der erste Abschnitt 155 umfasst eine Führungsstange 157 und einen Spindeltrieb 158. Der zweite Abschnitt 156 ist an dem ersten Abschnitt 155 gehalten. Er kann durch Bewegen einer Mutter 159 entsprechend dem Doppelpfeil 161 bewegt werden. An dem zweiten Abschnitt 156 ist die Ophthalmoskopierlupe 154 aufgenommen. Durch Bewegen der Mutter 159 lässt sich der Abstand der Ophthalmoskopierlupe 154 von einem Patientenauge 190, das untersucht werden soll, einstellen.

Die Ophthalmoskopierlupe 154 erzeugt ein Zwischenbild 191 des Hintergrunds 192 des Patientenauges 190. Ist das Mikroskop-Hauptobjektiv 102 auf das Zwischenbild 191 fokussiert, kann der Hintergrund 191 des Patientenauges 190 im Operationsmikroskop 100 scharf betrachtet werden.

Das Operationsmikroskop 100 hat ein Beleuchtungssystem 120 mit einer Lichtquelle 121. Die Lichtquelle stellt über eine Leuchtfeldblende 122 und Linsenelemente 123 und 124 Beleuchtungslicht 125 bereit, das auf ein Umlenkelement 126 gelangt. Das Umlenkelement 126 lenkt das Beleuchtungslicht 125 durch das Mikroskops-Hauptobjektivsystem 102 zum Patientenauge 190, dem Objektbereich.

Für das aus dem Mikroskop-Hauptobjektivsystem 102 austretende Beleuchtungslicht 125 ist im zweiten Abschnitt 156 der Halteeinrichtung 153 eine Linse 180 mit positiver Brechkraft vorgesehen. Diese Linse 180 wirkt als Beleuchtungslicht-Einkoppeleinheit. Sie fängt das Beleuchtungslicht 125, das durch das Mikroskop-Hauptobjektivsystem 102 gelangt, ein und leitet es zu einem Ablenkprisma 181. Das Ablenkprisma 181 führt das Beleuchtungslicht 125 an der Ophthalmoskopierlupe 154 seitlich vorbei und lenkt es zum Patientenauge 190. Das Vorbeiführen des Beleuchtungsstrahlenganges an der Ophthalmöskopierlupe 154 gewährleistet, dass keine störenden Reflexe von Beleuchtungslicht an der Ophthalmoskopierlupe 154 entstehen können, die dann wieder in dem Beobachtungsstrahlengang 106, 107 des Operationsmikroskops eingefangen werden.

Die Fig. 2 zeigt einen Schnitt des Ophthalmoskopie-Vorsatzmoduls 150 entlang der Linie II - II in Fig. 1. Der zweite Abschnitt 156 des Ophthalmoskopie-Vorsatzmoduls trägt an seinen zum Objektbereich weisenden Ende das Umlenkprisma 181 und die Ophthalmoskopierlupe 154 an einem Haltesteg 110.

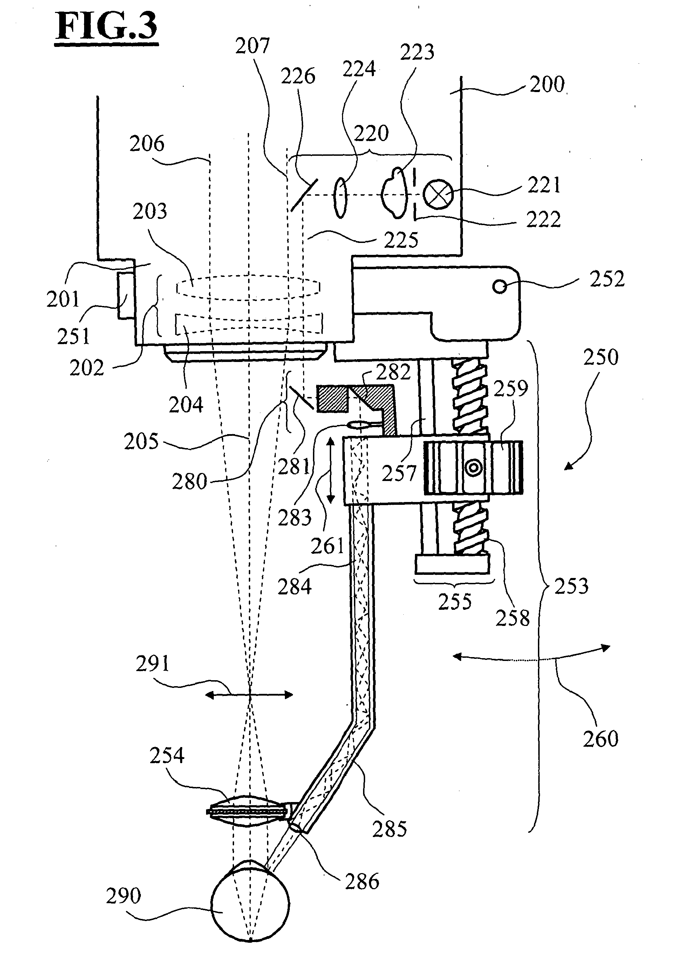

In Fig. 3 ist ein Operationsmikroskop 200 mit Ophthalmoskopie-Vorsatzmodul 250 gezeigt. Das Ophthalmoskopie-Vorsatzmodul 250 hat eine Halteeinrichtung 251, welche wiederum an einer ringförmigen Aufnahme 201 für Mikroskop-Hauptobjektivsystem 202 des Operationsmikroskops angeordnet ist. Das Mikroskop-Hauptobjektivsystem 202 ist fokussierbar gehalten und hat eine Linse 203 mit positiver Brechkraft und eine Linse 204, deren Brechkraft negativ ist. Das Ophthalmoskopie-Vorsatzmodul 250 hat eine Halteeinrichtung 253, die ein Gelenk 252 aufgenommen ist. Die Halteeinrichtung 253 trägt eine Ophthalmoskopierlupe 254. In dem Gelenk 252 kann die Ophthalmoskopierlupe 254 an der Halteeinrichtung 253 entsprechend dem Doppelpfeil 255 in und aus dem Beobachtungsstrahlengang 205 des Operationsmikroskops 200 geschwenkt werden.

Die Halteeinrichtung 253 hat wiederum einen ersten Abschnitt 255 und einen zweiten Abschnitt 256. Der erste Abschnitt 255 weist eine Führungsstange 257 und einen Spindeltrieb 258 auf. Der zweite Abschnitt 256 ist an dem ersten Abschnitt 255 gehalten. Er ist als Lichtleiter 284 ausgebildet und kann durch Bewegen der Mutter 259 entsprechend dem Doppelpfeil 260 bewegt werden.

In dem Operationsmikroskop 200 ist ein Beleuchtungssystem 220 vorgesehen, dessen Aufbau demjenigen des Beleuchtungssystems 120 im Operationsmikroskop 100 aus Fig. 1 entspricht. Die Baugruppen des Beleuchtungssystems, die schon in Fig. 1 gezeigt sind, tragen in Fig. 2 Bezugszeichen, die im Vergleich zu Fig. 1 um die Zahl 100 erhöht sind.

Das Operationsmikroskop 200 hat eine Beleuchtungslicht-Einkoppeleinheit 280, die einen Umlenkspiegel 281 und einen Umlenkspiegel 282 enthält. Ist die Halteeinrichtung 251, wie in Fig. 2 gezeigt, mit der Ophthalmoskopierlupe 254 in den Beobachtungsstrahlengang 206, 207 des Operationsmikroskops 200 geschwenkt, so wird das Beleuchtungslicht 225 aus dem Mikroskop-Hauptobjektivsystem vom Umlenkspiegel 281 eingefangen und über Umlenkspiegel 282 durch ein Linsenelement 283 in einen Lichtleiter 284 eingekoppelt. Der Lichtleiter 284 wirkt als Halteeinrichtung und trägt die Ophthalmoskopierlupe 254. Er ist in einem Abschnitt 255 der Halteeinrichtung 253 aufgenommen und kann durch Bewegen einer Mutter 258 derart verlagert werden, dass sich die Ophthalmoskopierlupe 254 entsprechend dem Doppelpfeil 260 heben und senken lässt. Der Lichtleiter 284 ist in einem zum Objektbereich weisenden Abschnitt 285 geknickt ausgeführt. Er leitet das durch ihn geleitete Beleuchtungslicht 225 durch ein Linsenelement 286, um so gebündelt auf das zu untersuchende Objekt in Form des Patientenauges 290 zu gelangen.

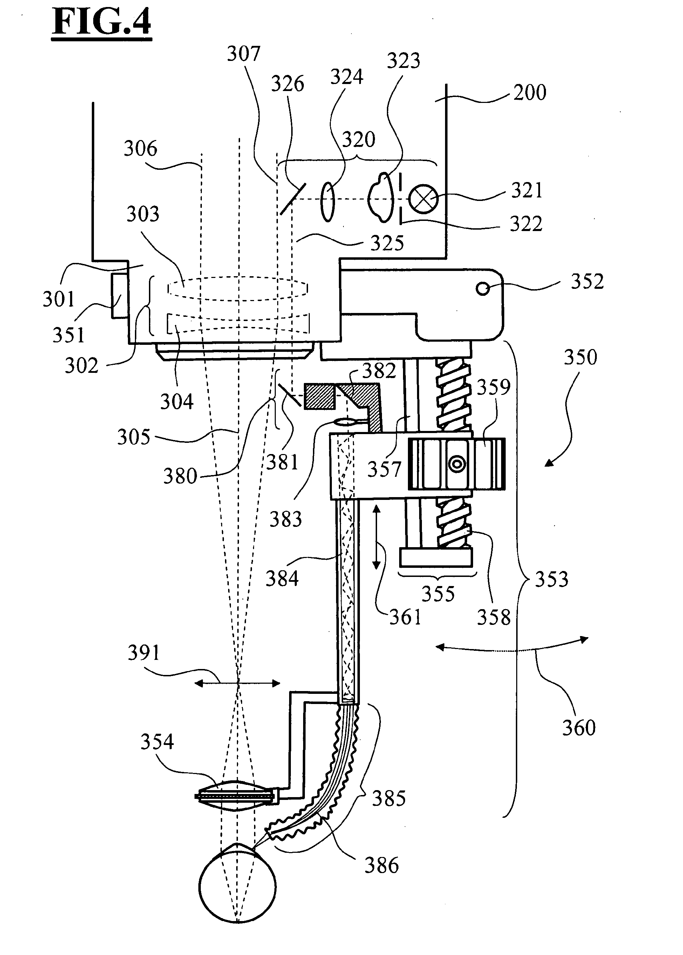

Die Fig. 4 zeigt ein Operationsmikroskop 300 mit Ophthalmoskopie-Vorsatzmodul 350, dessen Aufbau grundsätzlich dem Operationsmikroskop 200 mit Ophthalmoskopie-Vorsatzmodul 250 aus Fig. 3 entspricht. Baugruppen, die bei den in Fig. 3 und Fig. 4 gezeigten Operationsmikroskop mit Ophthalmoskopie-Vorsatzmodul identisch sind, tragen in Fig. 4 Bezugszeichen, die im Vergleich zu Fig. 3 um die Zahl 100 erhöht sind. Bei dem Ophthalmoskopie-Vorsatzmodul 350 ist ein Lichtleiter 384 vorgesehen, der einen schwanenhalsförmig ausgebildeten Abschnitt 385 aufweist. Dieser schwanenhalsförmig ausgebildete Abschnitt 385 enthält eine Faseroptik 386. Der Abschnitt 385 ist wie ein Gartenschlauch bewegbar. Sein Verlauf kann eingestellt werden. Der Abschnitt 385 behält dabei eine jede von einer Bedienperson einmal eingestellte Form bei.

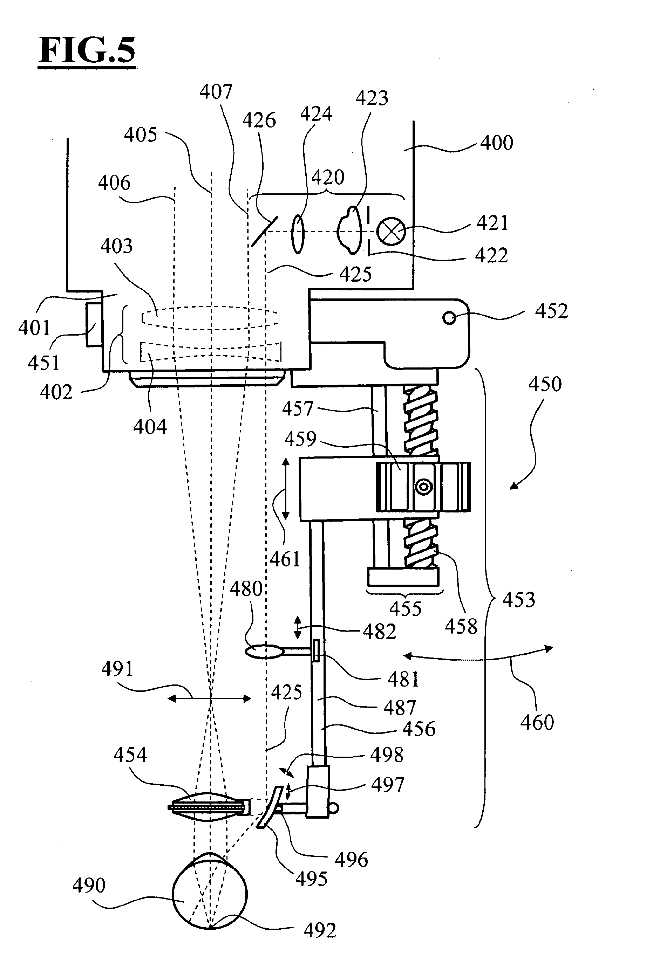

Die Fig. 5 zeigt ein Operationsmikroskop 400 mit Ophthalmoskopie-Vorsatzmodul 450. Der Aufbau des Operationsmikroskops 400 mit Ophthalmoskopie-Vorsatzmodul 450 entspricht demjenigen von Operationsmikroskop 100 und Ophthalmoskopie-Vorsatzmodul 150 aus Fig. 1. Einander entsprechende Baugruppen und Gegenstände sind daher im Vergleich zur Fig. 1 in Fig. 5 mit um die Zahl 300 erhöhten Bezugszeichen versehen.

Anders als das Ophthalmoskopie-Vorsatzmodul 150 aus Fig. 1 gibt es bei dem Ophthalmoskopie-Vorsatzmodul 450 kein Ablenkprisma sondern ein Spiegelelement 495 vorgesehen. Diesem Spiegelelement 495 ist ein Piezo-Antrieb 496 zugeordnet. Der Piezo-Antrieb ermöglicht, das Spiegelelement 495 entsprechend der Doppelpfeile 497 und 498 zu verstellen. Gleichzeitig ist dem Linsenelement 480 ein Antrieb 481 zugeordnet. Dieser Antrieb ermöglicht, das Linsenelement 480 entsprechend dem Doppelpfeil 482 am Abschnitt 487 der Halteeinrichtung 453 entlang zu bewegen.

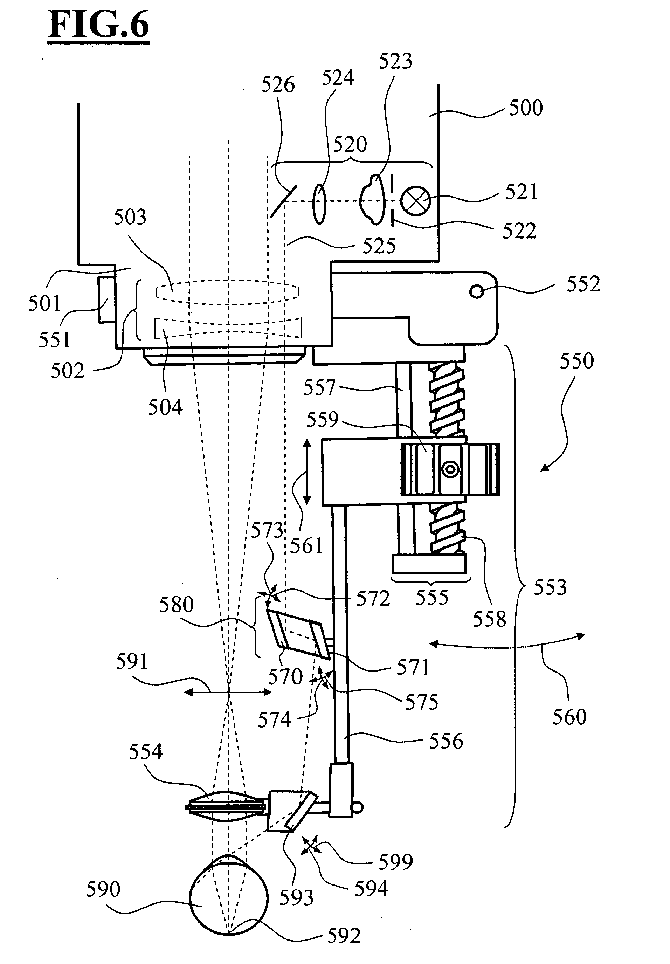

Die Fig. 6 zeigt ein Operationsmikroskop 500 mit einem Ophthalmoskopie-Vorsatzmodul 550, das ein Spiegelsystem aufweist, um Beleuchtungslicht 525, das mittels eines Beleuchtungssystems 520 im Operationsmikroskop 500 bereitgestellt wird, in ein zu untersuchendes Patientenauge 590 zu lenken. Soweit Baugruppen und Gegenstände in Fig. 5 und Fig. 6 identisch sind, tragen sie in Fig. 6 im Vergleich zu Fig. 5 um die Zahl 100 erhöhte Bezugszahlen.

Bei dem Ophthalmoskopie-Vorsatzmodul 550 ist ein erster gekrümmter Spiegel 570 und ein zweiter gekrümmter Spiegel 571 vorgesehen, die als Beleuchtungslicht-Einkoppeleinheit 580 wirken und das Beleuchtungslicht 525 einfängt, welches durch das Mikroskop-Hauptobjektivsystem 502 des Operationsmikroskops 500 tritt. Die Beleuchtungslicht-Einkoppeleinheit 580 ist entsprechend der Doppelpfeile 572, 573, 574, 575 verstellbar ausgeführt und wird an einem zweiten Abschnitt 556 der Halteeinrichtung 553 gehalten. Die Beleuchtungslicht-Einkoppeleinheit 580 lenkt Beleuchtungslicht 525 zum Umlenkspiegel 593, der entsprechend der Doppelpfeile 594, 599 verstellt werden kann. Dieser Umlenkspiegel 593 führt das Beleuchtungslicht an der Ophthalmoskopierlupe 554 vorbei zum Patientenauge 590.

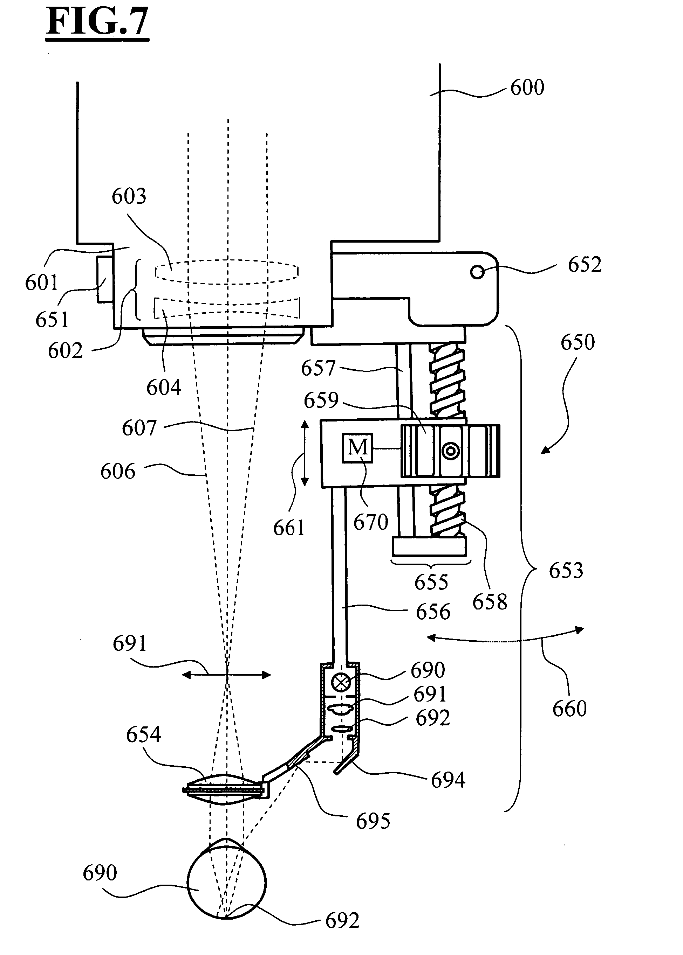

In Fig. 7 ist ein Operationsmikroskop 600 mit Ophthalmoskopie-Vorsatzmodul 650 gezeigt, bei dem wie beim Ophthalmoskopie-Vorsatzmodul 150 aus Fig. 1 das Ophthalmoskopie-Vorsatzmodul 650 mittels einer Halteeinrichtung 651 an einer Aufnahme 601 für ein fokussierbares Mikroskop-Hauptobjektivsystem 602 angeordnet ist. Das Mikroskop-Hauptobjektivssystem 602 hat eine Linse 603 mit positiver Brechkraft und eine Linse 604, deren Brechkraft negativ ist. Bei dem Ophthalmoskopie-Vorsatzmodul 650 ist ein Gelenk 652 vorgesehen. In dem Gelenk 652 ist eine Halteeinrichtung 653 gehalten, die eine Ophthalmoskopierlupe 654 trägt. Die Ophthalmoskopierlupe 654 kann wie die Ophthalmoskopierlupe 154 beim Ophthalmoskopie-Vorsatzmodul 150 aus Fig. 1 entsprechend dem Doppelpfeil 660 in und aus dem Beobachtungsstrahlengang 606, 607 des Operationsmikroskops 600 geschwenkt werden.

Die Halteeinrichtung 653 hat einen ersten Abschnitt 655 mit einer Führungsstange 657 und mit einer Gewindespindel 658, auf die eine Mutter 659 wirkt. Der erste Abschnitt 655 trägt einen zweiten Abschnitt 656, an welchem die Ophthalmoskopierlupe 654 aufgenommen ist. Der Mutter 659 ist ein Verstellmotor 670 zugeordnet. Mittels des Verstellmotors 670 kann die Mutter 659 steuerbar angetrieben werden, wodurch der zweite Abschnitt 656 der Halteeinrichtung 653 relativ zu dem ersten Abschnitt 655 entsprechend des Doppelpfeils 661 bewegt werden kann. In dem zweiten Abschnitt 656 ist eine Lichtquelle 690 mit Beleuchtungsoptik integriert. Diese Lichtquelle umfasst Linsenelemente 691 und 692. Die Lichtquelle 690 ist über nicht weiter dargestellte Zuleitungen aus dem Operationsmikroskop 600 mit elektrischer Energie versorgt. Alternativ ist es auch möglich, für die Lichtquelle eine Batteriespeisung vorzusehen.

Das von der Lichtquelle 690 bereitgestellte Beleuchtungslicht 625 wird, nachdem es durch die Beleuchtungsoptik geführt ist, auf Spiegelflächen 694 und 695 geleitet. Diese Spiegelflächen 694, 695 lenken das Beleuchtungslicht 625 an der Ophthalmoskopierlupe 654 vorbei zum Objektbereich in Form des Patientenauges 690.

## Patentansprüche

1. Ophthalmoskopie-Vorsatzmodul (150, 250, 350, 450, 550, 650) zur Befestigung an einem Operationsmikroskop (100, 200, 300, 400, 500, 600)
- mit einer Halteeinrichtung (153, 253, 353, 453, 553, 653) für eine Ophthalmoskopierlupe (154, 254, 354, 454, 554, 654); wobei
- die Halteeinrichtung (153, 253, 353, 453, 553, 653) eine Einrichtung (180, 181, 281, 282, 283, 284, 285, 286, 381, 382, 383, 384, 385, 480, 495, 570, 571, 593, 691, 692, 694, 695) zum Führen von Beleuchtungslicht trägt;
**dadurch gekennzeichnet, dass**
- die Einrichtung zum Führen von Beleuchtungslicht (180, 181, 281, 282, 283, 284, *285, 286,* 381, 382, 383, 384, 385, 480, 495, 570, 571, 593, 691, 692, 694, 695) Beleuchtungslicht (125, 225, 325, 425, 525, 625) an der Ophthalmoskopierlupe (154, 254, 354, 454, 554, 654) vorbei zum Objektbereich (190, 290, 390, 490, 590, 690) führt.

2. Ophthalmoskopie-Vorsatzmodul nach Anspruch 1, **dadurch gekennzeichnet, dass** die Einrichtung (385, 480, 495, 570, 571, 593) zum Führen von Beleuchtungslicht (325, 425, 525) wenigstens teilweise verstellbare optische Elemente (386, 480, 495, 570, 571, 593) umfasst.

3. Ophthalmoskopie-Vorsatzmodul nach Anspruch 2, **dadurch gekennzeichnet, dass** den wenigstens teilweise verstellbaren optischen Elementen Antriebe (481, 496) zugeordnet sind.

4. Ophthalmoskopie-Vorsatzmodul nach einem der Ansprüche 1 bis 3, **dadurch gekennzeichnet, dass** die Einrichtung zum Führen von Beleuchtungslicht eine oder mehrere Spiegel (281, 282, 381, 384, 495, 570, 571, 593) umfasst.

5. Ophthalmoskopie-Vorsatzmodul nach einem der Ansprüche 1 bis 4, **dadurch gekennzeichnet, dass** die Einrichtung zum Führen von Beleuchtungslicht eine oder mehrere Lichtleiter (284, 384, 385) umfasst.

6. Ophthalmoskopie-Vorsatzmodul nach einem der Ansprüche 1 bis 5, **dadurch gekennzeichnet, dass** die Einrichtung zum Führen von Beleuchtungslicht ein oder mehrere Linsenelemente (180, 283, 286, 383, 480) umfasst.

7. Ophthalmoskopie-Vorsatzmodul nach einem der Ansprüche 1 bis 6, **dadurch gekennzeichnet, dass** die Einrichtung zum Führen von Beleuchtungslicht ein Ablenkprisma (181) umfasst.

8. Ophthalmoskopie-Vorsatzmodul nach einem der Ansprüche 1 bis 7, **dadurch gekennzeichnet, dass** in das Ophthalmoskopie-Vorsatzmodul (650) eine Lichtquelle (690) integriert ist.

9. Ophthalmoskopie-Vorsatzmodul nach einem der Ansprüche 1 bis 8, **dadurch gekennzeichnet, dass** eine Beleuchtungslicht-Einkoppeleinheit (180, 280, 380, 480, 580) vorgesehen ist, um von dem Operationsmikroskop (100, 200, 300, 400, 500) bereitgestelltes Beleuchtungslicht (125, 225, 325, 425, 525) aufzunehmen.

10. Ophthalmoskopie-Vorsatzmodul nach Anspruch 9, **dadurch gekennzeichnet, dass** die Beleuchtungslicht-Einkoppeleinheit (180, 280, 380, 480, 580) zur Aufnahme von Beleuchtungslicht (125, 225, 325, 425, 525) ausgelegt ist, welches das Mikroskop-Hauptobjektiv (102, 202, 302, 402, 502) durchsetzt.

11. Operationsmikroskop (100, 200, 300, 400, 500, 600) mit einem Ophthalmoskopie-Vorsatzmodul (150, 250, 350, 450, 550, 660) gemäß einem der Ansprüche 1 bis 10.
